# DEMANDE DE BREVET EUROPEEN

(11) **EP 1 674 076 A2**
(43) Date de publication de la demande: **28.06.2006**
(21) Numéro de dépôt: 05301066.6
(22) Date de dépôt: 16.12.2005
(51) Int. Cl.: A61K 8/81, A61K 8/73, A61Q 1/10

(54) **Composition cosmétique pour le maquillage résistante à l'eau et facilement démaquillable**

(30) Priorité: 21.12.2004 FR 0453120
(71) Demandeur: L'ORÉAL, 75008 Paris (FR)
(72) Inventeur: Pays, Karl, 94410 Saint-Maurice (FR); Raineau, Olivier, 75005 Paris (FR); Bichon, Yohann, 94700 Maison-Alfort (FR)
(74) Mandataire: Le Coupanec, Pascale A.M.P.

(57) **Abrégé**

La présente invention concerne une composition cosmétique pour le maquillage des matières kératiniques, ladite composition ayant une phase continue huileuse et comportant au moins un polyélectrolyte, au moins un agent tensioactif de HLB supérieur ou égal à 6 à 25 °C et au moins une matière colorante, caractérisée en ce que le polyélectrolyte est branché et/ou réticulé et en ce que la teneur en eau et/ou en solvant hydrosoluble éventuellement présente est inférieure à 50 % en poids par rapport au poids total de la composition. Elle concerne aussi une Composition cosmétique non rincée pour le maquillage et/ou le soin non thérapeutique des fibres kératiniques, ladite composition ayant une phase continue huileuse et comportant au moins un polyélectrolyte et au moins un agent tensioactif de HLB supérieur ou égal à 6 à 25 °C, caractérisée en ce que le polyélectrolyte est branché et/ou réticulé et en ce que la teneur en eau et/ou en solvant hydrosoluble éventuellement présente est inférieure à 50 % en poids par rapport au poids total de la composition. Elle concerne encore une Composition cosmétique non rincée pour le maquillage et/ou le soin non thérapeutique des fibres kératiniques, ladite composition ayant une phase continue huileuse et comportant au moins un polyélectrolyte et au moins un agent tensioactif de HLB supérieur ou égal à 6 à 25 °C, caractérisée en ce que le polyélectrolyte est choisi parmi un copolymère acrylamide/acide 2-méthyl 2-[(1-oxo-2-propényl)amino]1- propanesulfonique, un glycolate d'amidon réticulé sous forme de poudre, un polyacrylate, un copolymère greffé à base d'amidon, les dérivés ionisables de polysaccharides, les acides polyacryliques, les copolymères acide polyacryliques acrylates d'alkyle, l'AMPS (Acide polyacrylamidométhyl propane sulfonique neutralisé partiellement à l'ammoniaque et hautement réticulé), les copolymères AMPS/méthacrylates d'alkyle polyoxyéthylénés (réticulés ou non), et un mélange de ces derniers et de préférence parmi un copolymère acrylamide/acide 2-méthyl 2-[(1-oxo-2-propényl)amino]1-propanesulfonique, un polyacrylate, et leurs copolymères.
La présente invention concerne en outre l'utilisation de l'association d'au moins un polyélectrolyte et d'au moins un agent tensioactif de HLB supérieur ou égal à 6 à 25 °C, pour la préparation d'une composition cosmétique pour le maquillage et/ou le soin non thérapeutique des matières kératiniques à phase continue huileuse, présentant une bonne tenue à l'eau et une facilité de démaquillage, ainsi que le procédé cosmétique associé.

## Description

La présente invention concerne des compositions cosmétiques pour le maquillage et/ou le soin non thérapeutique des matières kératiniques notamment de la peau, des lèvres et/ou des fibres kératiniques, susceptibles de résister à l'eau et d'être facilement démaquillées.

Plus particulièrement, les compositions selon l'invention, peuvent constituer un produit de maquillage du visage, du corps et/ou des lèvres et des fibres kératiniques, comme les cils, les sourcils et les cheveux, et plus particulièrement un produit de maquillage des cils.

Il peut notamment s'agir d'une composition de maquillage, d'une composition transparente ou colorée à appliquer sur ou sous un maquillage, dites encore respectivement « top-coat » ou « base-coat », ou bien encore d'une composition de traitement des cils.

La composition selon l'invention peut se présenter sous la forme d'un produit pour les cils ou mascara, d'un produit pour les sourcils, ou d'un produit de maquillage des cheveux. Plus spécialement, l'invention porte sur un mascara.

Il existe en pratique essentiellement deux types de formulation de mascara, à savoir d'une part, des mascaras à phase continue aqueuse, dits « mascaras émulsion », se présentant sous forme d'émulsion de cires dans l'eau et, d'autre part, des mascaras à phase continue solvant ou huile, anhydres ou à faible teneur en eau et/ou solvants hydrosolubles, dits «mascaras waterproof », formulés à l'état de dispersion, dans des solvants non aqueux, d'au moins un structurant huileux qui peut être une cire, un polymère, en particulier un polymère semi-cristallin ou un gélifiant lipophile.

La présente invention est relative plus particulièrement aux compositions ou mascaras de type « waterproof » tels que définis ci-dessus.

Ces mascaras dits « waterproof » sont connus pour donner lieu à un démaquillage difficile, voire impossible avec certains démaquillants, en particulier les démaquillants principalement aqueux ou hydrosolubles, notamment les solutions aqueuses. Le démaquillage s'effectue donc en règle générale à l'aide de démaquillants spécifiques à base d'huiles ou de solvants organiques. Or ces démaquillants peuvent être irritants pour l'oeil, notamment provoquer des picotements ou laisser un voile sur l'oeil, ou bien encore peuvent laisser sur la peau autour de l'oeil (paupières) un film résiduel gras inconfortable.

Il existe donc un besoin de disposer de compositions cosmétiques pour le maquillage susceptible à la fois de présenter une bonne tenue à l'eau et à la fois une bonne aptitude au démaquillage, y compris avec les démaquillants usuels.

Les documents FR 2 785 801, EP 1 152 022, FR 2 774 996, WO 95/35089 et WO 99/26445 décrivent des compositions épaississantes, dites « latex épaississant » ou « agent épaississant » ou encore « latex inverse ».

Les documents FR 2 785 801 et FR 2 774 996 divulguent notamment des compositions comprenant une phase aqueuse, une phase huileuse, un agent émulsifiant de type H/E (huile dans eau) et un agent émulsifiant de type E/H (eau dans huile) ainsi qu'un polyélectrolyte anionique, branché ou réticulé, à base d'un monomère possédant une fonction acide fort.

Le document WO 99/52499 décrit principalement des rouges à lèvres comprenant un polyacrylate de sodium dans le but de produire un effet volumateur.

On a maintenant découvert qu'il était possible d'obtenir des compositions cosmétiques pour le maquillage et/ou le soin non thérapeutique des matières kératiniques capables de concilier une bonne tenue à l'eau et un démaquillage aisé quel que soit le type de démaquillant.

Selon un premier aspect, la présente invention concerne une composition cosmétique pour le maquillage des matières kératiniques, ladite composition ayant une phase continue huileuse et comportant au moins un polyélectrolyte, au moins un agent tensioactif de HLB supérieur ou égal à 6 à 25 °C et au moins une matière colorante, caractérisée en ce que le polyélectrolyte est branché et/ou réticulé et en ce que la teneur en eau et/ou en solvant hydrosoluble éventuellement présente est inférieure à 50 % en poids par rapport au poids total de la composition.

Les compositions selon l'invention peuvent notamment être des compositions cosmétiques non rincées. Ainsi, les compositions selon l'invention sont généralement différentes des solutions de démaquillage nécessitant, après usage, un rinçage consécutif visant à les éliminer totalement de la matière kératinique considérée.

La présente invention est plus particulièrement relative à des compositions destinées à être appliquées sur les matières kératiniques, et de préférence sur les fibres kératiniques.

Ainsi, selon un deuxième aspect, la présente invention concerne une composition cosmétique non rincée pour le maquillage et/ou le soin des fibres kératiniques, ladite composition ayant une phase continue huileuse et comportant au moins un polyélectrolyte et au moins un agent tensioactif de HLB supérieur ou égal à 6 à 25 °C, caractérisée en ce que le polyélectrolyte est branché et/ou réticulé et en ce que la teneur en eau et/ou en solvant hydrosoluble éventuellement présente est inférieure à 50 % en poids par rapport au poids total de la composition.

Selon un troisième aspect, la présente invention concerne une composition cosmétique non rincée pour le maquillage et/ou le soin non thérapeutique des fibres kératiniques, ladite composition ayant une phase continue huileuse et comportant au moins un polyélectrolyte et au moins un agent tensioactif de HLB supérieur ou égal à 6 à 25 °C, caractérisée en ce que le polyélectrolyte est choisi parmi un copolymère acrylamide/acide 2-méthyl 2-[(1-oxo-2-propényl)amino]1-propanesulfonique, un glycolate d'amidon réticulé sous forme de poudre, un polyacrylate, un copolymère greffé à base d'amidon, les dérivés ionisables de polysaccharides, les acides polyacryliques, les copolymères acide polyacryliques acrylates d'alkyle, l'AMPS (Acide polyacrylamidométhyl propane sulfonique neutralisé partiellement à l'ammoniaque et hautement réticulé), les copolymères AMPS/méthacrylates d'alkyle polyoxyéthylénés (réticulés ou non), et un mélange de ces derniers et de préférence parmi un copolymère acrylamide/acide 2-méthyl 2-[(1-oxo-2-propényl)amino] 1-propanesulfonique, un polyacrylate, et leurs copolymères.

Selon un quatrième aspect, la présente invention concerne un procédé cosmétique de maquillage des matières kératiniques comprenant au moins une étape d'application sur les matières kératiniques d'une composition telle que définie précédemment.

Selon un cinquième aspect, la présente invention concerne un procédé cosmétique de maquillage et/ou de soin non thérapeutique des fibres kératiniques comprenant au moins une étape d'application sur les fibres kératiniques d'une composition non rincée telle que définie précédemment.

Selon un sixième aspect, la présente invention a pour objet un support maquillé comprenant un maquillage susceptible d'être obtenu selon l'un quelconque des procédés tels que définis précédemment.

Selon un septième aspect, la présente invention concerne l'utilisation de l'association d'au moins un polyélectrolyte et d'au moins un agent tensioactif de HLB supérieur ou égal à 6 à 25 °C, pour la préparation d'une composition cosmétique pour le maquillage des matières kératiniques à phase continue huileuse, présentant une bonne tenue à l'eau et une facilité de démaquillage.

Selon un huitième aspect, la présente invention concerne l'utilisation de l'association d'au moins un polyélectrolyte et d'au moins un agent tensioactif de HLB supérieur ou égal à 6 à 25 °C, pour la préparation d'une composition cosmétique non rincée pour le maquillage et/ou le soin non thérapeutique des fibres kératiniques à phase continue huileuse, présentant une bonne tenue à l'eau et une facilité de démaquillage.

Comme il ressort des exemples ci-après, les compositions selon l'invention manifestent de tels avantages techniques.

Par « composition cosmétique à phase continue huileuse », on entend un système apte à se diluer ou se disperser au contact dudit milieu solvant ou huile.

Par « huile ou solvant organique volatile », on entend une huile ou solvant organique (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 8000 Pa (0,01 à 60 mm de Hg).

Dans le cadre de la présente invention, le terme « matières kératiniques » comprend la peau, les lèvres, les ongles, les cheveux, les cils et les sourcils.

Dans le cadre de la présente invention, par « fibres kératiniques » on entend notamment les cheveux, les cils et les sourcils. De plus, le maquillage de la peau s'entend notamment du maquillage du corps, des mains, du cou ou du visage.

La composition selon l'invention comprend un milieu physiologiquement acceptable, notamment cosmétiquement acceptable, c'est-à-dire un milieu compatible en particulier avec les matières kératiniques et notamment les fibres kératiniques telles que les cheveux, les cils, les sourcils.

Par « cosmétiquement acceptable », on entend, dans le cadre de la présente invention, un composé dont l'utilisation est compatible avec une application sur les matières kératiniques.

D'une manière générale, dans un souci de simplification, et sauf indication contraire, les teneurs sont indiquées en matière sèche.

### POLYELECTROLYTE

Par « polyélectrolyte », on entend une substance macromoléculaire, qui a la faculté de se dissocier, lorsqu'elle est dissoute dans l'eau ou dans tout autre milieu ionisant, pour donner au moins un ion. Autrement dit, un polyélectrolyte est un polymère comportant au moins un monomère ionisable.

En particulier, le polyélectrolyte peut donner des polyions, par exemple des polyanions, lorsqu'il est dissocié dans l'eau. Un polyélectrolyte peut être un polyacide, une polybase, un polysel ou polyampholyte. Dans le cadre de l'invention, il est de préférence un polyacide et de préférence un polyacide fort.

De façon préférée, le polyélectrolyte compris dans les compositions cosmétiques selon la présente invention est un polymère anionique branché et/ou réticulé.

De préférence, le polyélectrolyte est en outre apte à former en solution aqueuse, au-delà d'une concentration supérieure ou égale à 0,1 % en poids de matière sèche, de préférence ≥ 0,3 % en poids par rapport au poids total de la composition, un gel. Ce gel peut être caractérisé par rhéologie oscillatoire (v = 1 Hz) par un seuil d'écoulement τ_{c} au moins égal à 10 Pa.

De plus, lorsque la composition cosmétique selon la présente invention comprend un polymère filmogène, le polyélectrolyte se distingue avantageusement de ce polymère filmogène.

Les contre-ions des polyions formés lors de la dissociation peuvent être de toute nature, inorganique ou organique.

En particulier, lorsque le polyélectrolyte est un polymère anionique branché ou réticulé, les cations peuvent être des cations alcalins ou alcalino-terreux tels que le sodium ou le potassium ou encore l'ion ammonium.

Le cation sodium Na⁺ est préféré, c'est pourquoi il est principalement cité dans la liste de polyélectrolytes qui va suivre, sans que cela constitue une quelconque limitation à ce contre-ion spécifique.

A titre de polyélectrolyte, on peut citer :
- le copolymère acrylamide / AMPS de Na tel que le Simulgel 600® sous forme d'émulsion contenant du polysorbate 80 à titre de tensioactif et contenant de l'isohexadécane à titre de phase huile, vendu par la société SEPPIC, ou encore le Simulgel EG® , le Simulgel A® et le Simulgel 501® vendus par la même société.
   Le Simulgel 600® est notamment décrit dans le document FR 2 785 801. Il s'agit en réalité d'un latex inverse. Le polyélectrolyte AMPS est de l'acide 2-méthyl 2-[(1-oxo 2-propényl)amino] 1-propanesulfonique partiellement ou totalement salifié notamment sous forme de sel de sodium ou de sel d'ammonium compris à hauteur de 30 à 50 % en proportions molaires dans le mélange comprenant de l'AMPS ainsi qu'un acrylamide, contenu quant à lui à hauteur de 50 à 70 %.
- le glycolate sodique d'amidon réticulé sous forme de poudre,
- les polyacrylates de sodium tel que le Norsocryl S35® vendu par la société ATOFINA, ou le Cosmedia SP® vendu par la société COGNIS,
- les dérivés ionisables de polysaccharides tels que les sels de cellulose et les alginates de sodium,
- les copolymères greffés à base d'amidon tels que les Waterlock® (A-180 et G-400 par exemple) de chez Grain Processing Corporation,
- les acides polyacryliques de type SYNTHLEN K® ,
- les copolymères acide polyacryliques acrylates d'alkyle de type PEMULEN® ,
- l'AMPS (Acide polyacrylamidométhyl propane sulfonique neutralisé partiellement à l'ammoniaque et hautement réticulé) par exemple commercialisé par la société CLARIANT,
- les copolymères AMPS/méthacrylates d'alkyle polyoxyéthylénés (réticulés ou non),
- le carboxy méthyl cellulose sodique et tous les dérivés ionisables de la cellulose, et
- leurs mélanges.

Conviennent tout particulièrement à l'invention le polyacrylate de sodium et le copolymère acrylamide/AMPS et leurs copolymères.

Il sera bien entendu fait en sorte que la teneur en polyélectrolyte soit ajustée de telle manière que l'aptitude au démaquillage soit effectivement améliorée tout en étant non préjudiciable à la tenue à l'eau de la composition cosmétique.

Il est entendu que la quantité en polyélectrolyte est susceptible de varier significativement selon la nature du polyélectrolyte. De manière générale, cette quantité est au moins égale à la quantité nécessaire et suffisante pour conférer à ladite composition une meilleure aptitude au démaquillage. Elle est encore qualifiée de quantité efficace.

Cette aptitude au démaquillage peut notamment être appréciée à l'aide du test figurant dans les exemples ci-après.

Selon un mode de réalisation préféré de l'invention, le polyélectrolyte est présent dans une teneur en poids par rapport au poids total de la composition supérieure ou égale à 0,05 %, et de façon préférée supérieure ou égale à 0,1 %, et notamment supérieure ou égale à 0,5 %.

Selon une variante préférée, le polyélectrolyte est présent dans la composition cosmétique de préférence dans une teneur allant de 0,05 à 15 % en poids, de façon encore plus préférée de 0,1 à 10 % en poids, et mieux encore de 0,5 à 5 % en poids par rapport au poids total de la composition.

Sans que cela constitue une quelconque limitation à l'invention, les inventeurs ont émis l'hypothèse que le polyélectrolyte en dispersion dans l'huile joue le rôle de « pompe à eau ». Ainsi, ce rôle de « pompe à eau » apparaît plus nettement lorsque la composition est mise en contact avec une phase aqueuse. Du fait de la présence de la pression osmotique élevée dans les réservoirs aqueux contenant le polyélectrolyte en raison de la présence de contre-ions, lesdits réservoirs gonflent jusqu'à l'obtention d'une inversion de phase.

Ainsi au niveau macroscopique, lors du démaquillage, le film de la composition cosmétique selon l'invention, s'hydrate grâce aux nombreuses « pompes à eau » microdispersées dans le film. Ledit film se fragilise et entraîne sa rupture mécanique. Il y a alors morcellement du film.

La présence de l'agent tensioactif d'HLB supérieur ou égal à 6 à 25 °C permet de stabiliser au final la composition cosmétique, une fois hydratée c'est-à-dire sous forme d'une émulsion à phase continue aqueuse, autrement dit, sous forme d'une émulsion huile-dans-eau.

### TENSIOACTIF

La composition selon l'invention, contient au moins un agent tensioactif de HLB supérieur ou égal à 6 à 25 °C.

Selon un mode de réalisation particulier, il est ou ils sont présent(s) dans une teneur en poids supérieure ou égale à 0,1 % par rapport au poids total de la composition. Il(s) peu(ven)t être présent(s) notamment en une proportion allant de 0,1 à 30 %, mieux de 0,5 à 15 % et mieux de 1,5 à 10 % en poids par rapport au poids total de la composition.

Il est entendu que lorsque le polyélectrolyte est incorporé à la composition de l'invention sous forme d'une composition déjà formulée avec un agent tensioactif de HLB supérieur ou égal à 6 à 25 °C, la quantité en agent tensioactif ci-dessus définie tient compte de la teneur en ledit agent tensioactif comprise dans la formulation du polyélectrolyte.

Le « HLB supérieur ou égal à 6 » s'entend d'un tensioactif possédant à 25 °C une balance HLB (hydrophile-lipophile-balance) au sens de GRIFFIN supérieure ou égale à 6.

La valeur HLB selon GRIFFIN est défmie dans J. Soc. Cosm. Chem. 1954 (volume 5), pages 249-256.

On peut se reporter au document « Encyclopedia of Chemical Technology, KIRK-OTHMER », volume 22, p. 333-432, 3^{ème} édition, 1979, WILEY, pour la défmition des propriétés et des fonctions (émulsionnant) des tensioactifs, en particulier p. 347-377 de cette référence, pour les tensioactifs non ioniques.

L'agent tensioactif de HLB supérieur ou égal à 6 à 25 °C compris dans la composition cosmétique selon la présente invention peut être ionique, non ionique ou à caractère mixte ionique et non ionique.

Parmi les agents tensioactifs non ioniques de HLB supérieur ou égal à 6 à 25 °C pouvant être présents dans les compositions selon la présente invention, utilisés seuls ou en mélange; on peut citer notamment :
- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C₈-C₂₄, et de préférence en C₁₂-C₁₈) tels que l'éther oxyéthyléné de l'alcool cétéarylique à 30 groupes oxyéthylénés (nom CTFA « Ceteareth-30 ») et l'éther oxyéthyléné du mélange d'alcools gras en C₁₂-C₁₅ comportant 7 groupes oxyéthylénés (nom CTFA « C12-15 Pareth-7 ») par exemple commercialisé sous la dénomination NEODOL 25-7® par SHELL CHEMICALS,
- les esters d'acide gras (notamment d'acide en C₈-C₂₄, et de préférence en C₁₆-C₂₂) et de polyéthylène glycol (pouvant comprendre de 1 à 150 motifs d'éthylèneglycol) tels que le stéarate de PEG-50 et le monostéarate de PEG-40 par exemple commercialisé sous le nom MYRJ 52P® par la société ICI UNIQUEMA,
- les esters d'acide gras (notamment d'acide en C₈-C₂₄, et de préférence en C₁₆-C₂₂) et des éthers de glycérol oxyéthylénés et/ou oxypropylénés (pouvant comporter de 1 à 150 groupes oxyéthylénés et/ou oxypropylénés), comme le monostéarate de PEG-200 glycéryle par exemple vendu sous la dénomination Simulsol 220 TM® par la société SEPPIC ; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,
- les esters d'acide gras (notamment d'acide en C₈-C₂₄, et de préférence en C₁₆-C₂₂) et des éthers de sorbitol oxyéthylénés et/ou oxypropylénés (pouvant comporter de 1 à 150 groupes oxyéthylénés et/ou oxypropylénés), comme le polysorbate 60 par exemple vendu sous la dénomination Tween 60® par la société UNIQUEMA, ainsi que le polysorbate 80, le polysorbate 40 et le polysorbate 20.
- la diméthicone copolyol, telle que celle vendue sous la dénomination Q2-5220® par la société DOW CORNING,
- la diméthicone copolyol benzoate (FINSOLV SLB 101® et 201® de la société FINTEX),
- les copolymères d'oxyde propylène et d'oxyde d'éthylène, également appelés polycondensats OE/OP,
- et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

H-(O-CH₂-CH₂)ₐ-(O-CH(CH₃)-CH₂)_{b}-(O-CH₂-CH₂)ₐ-OH,

formule dans laquelle a va de 2 à 120, et b va de 1 à 100.

Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol par exemple vendus sous les dénominations SYNPERONIC® comme les SYNPERONIC PE/ L44® et SYNPERONIC PE/F127® par la société ICI.

Parmi les agents tensioactifs ioniques, qui peuvent être anioniques ou cationiques, de HLB supérieur ou égal à 6 à 25 °C, pouvant être présents dans la composition selon la présente invention, utilisés seuls ou en mélange, on peut citer notamment :
- les tensioactifs siliconés comme les diméthicones copolyols phosphates tels que celui vendu sous la dénomination PECOSIL PS 100® par la société PHOENIX CHEMICAL,
- les dérivés d'acide aminés, tels que le lauryl sarcosinate, et le lauryl taurate,
- les sels d'acides gras en C₁₆-C₃₀, notamment ceux dérivant des amines, comme le stéarate de triéthanolamine,
- les sels d'acides gras polyoxyéthylénés, notamment ceux dérivant des amines ou les sels alcalins, et leurs mélanges,
- les esters phosphoriques et leurs sels tels que le «DEA oleth-10 phosphate » (Crofados N 10N® de la société CRODA),
- les sulfosuccinates tels que le « Disodium PEG-5 citrate lauryl sulfosuccinate » et le « Disodium ricinoleamido MEA sulfosuccinate »,
- les alkyléthersulfates tels que le lauryl éther sulfate de sodium,
- les iséthionates,
- les acylglutamates tels que le « Disodium hydrogenated tallow glutamate » (AMISOFT HS-21 R® commercialisé par la société AJINOMOTO) et leurs mélanges.

Convient tout particulièrement à l'invention le stéarate de triéthanolamine. Ce dernier est généralement obtenu par simple mélange de l'acide stéarique et de la triéthanolamine.

A titre représentatif des tensioactifs cationiques, on peut notamment citer :
- les alkyl-imidazolidinium tels que l'étho-sulfate d'isostéaryl-éthylimidonium,
- Les sels d'ammonium tels que le chlorure de N,N,N,-triméthyl-1-docosanamimium (chlorure de Behentrimonium).

Les compositions selon l'invention peuvent également contenir un ou plusieurs tensioactifs amphotériques comme les N-acyl-aminoacides, tels que les N-alkyl-aminoacétates et le cocoamphodiacétate.

Il serait bien entendu fait en sorte que l'agent tensioactif de HLB supérieur ou égale à 6 à 25 °C soit présent dans une quantité telle que la composition se présente bien sous forme d'une émulsion à phase continue huileuse.

Les agents tensioactifs non ioniques et ioniques de HLB supérieur ou égal à 6 à 25 °C décrits ci-dessus peuvent être également présents en association.

Par ailleurs, les compositions cosmétiques selon la présente invention comprennent de préférence un ou des agent(s) tensioactif(s) de HLB supérieur ou égal à 10 à 25 °C, et de façon encore plus préférée de HLB supérieur ou égal à 12.

La composition de l'invention, dans la mesure où il s'agit d'une composition à phase continue huileuse peut contenir également un agent tensioactif de bas HLB, à savoir inférieur à 6 parmi lesquels on peut citer :
a) les agents tensioactifs non ioniques de HLB inférieur à 6 à 25 °C, tels que :
   - les esters et éthers d'oses tels que les stéarate de sucrose, cocoate de sucrose, stéarate de sorbitan et leurs mélanges comme l'Arlatone 2121® commercialisé par la société ICI ;
   - les esters d'acides gras (notamment d'acide en C₈-C₂₄, et de préférence en C₁₆-C₂₂) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
   - le mélange de cyclométhicone/diméthicone copolyol vendu sous la dénomination Q2-3225C® par la société DOW CORNING,
   - les alcools gras tels que l'alcool cétylique, l'alcool stéarique,
   - les alcools gras éthoxylés avec un nombre d'éthoxylation tel que le HLB est inférieur à 6.

Conviennent tout particulièrement à l'invention, à titre d'agent tensioactif de HLB supérieur ou égal à 6 à 25 °C, les esters d'acide gras et des éthers de sorbitol oxyéthylénés et/ou oxypropylénés (pouvant comporter de 1 à 150 groupes oxyéthylénés) comme le polysorbate 20 de HLB 16,7, le polysorbate 40 de HLB 15,6, le polysorbate 60 de HLB 14,9 et le polysorbate 80 de HLB 15,0.

La composition selon l'invention peut comprendre d'autre(s) tensioactif(s) qui sont par exemple introduits dans la composition par l'introduction de la dispersion aqueuse de particules d'un polymère, ces tensioactifs étant ceux classiquement utilisés pour les stabiliser.

### HUILES

La composition selon l'invention comprend au moins une huile ou un solvant organique. Il peut s'agir d'un mélange d'huiles ou de solvants organiques.

La composition selon l'invention comprend habituellement une quantité d'huile totale variant de 10 à 90 %, de préférence de 15 à 80 % et mieux de 20 à 60 % en poids par rapport au poids total de la composition, que l'on peut aussi nommer milieu solvant non aqueux.

La ou les huiles présente(s) dans la composition de l'invention peu(ven)t être choisie(s) parmi les huiles volatiles et/ou les huiles non volatiles, et leurs mélanges.

On préfère, dans le cadre de la présente invention, les compositions cosmétiques comprenant essentiellement des huiles volatiles.

Par « huile ou solvant organique volatile », on entend une huile ou solvant organique (ou milieu non aqueux) susceptible de s'évaporer au contact de la peau en moins d'une heure, à température ambiante et pression atmosphérique.

L'huile volatile est une huile cosmétique volatile, liquide à température ambiante, ayant notamment une pression de vapeur non nulle, à température ambiante et pression atmosphérique, en particulier ayant une pression de valeur allant de 0,13 Pa à 40 000 Pa (10⁻³ à 300 mm de Hg), et de préférence allant de 1,3 Pa à 8 000 Pa ( 0,01 à 60 mm de Hg).

Les huiles (ou solvants organiques) volatiles peuvent être des huiles hydrocarbonées, des huiles siliconées, des huiles fluorées ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux « d'Isopars® » ou de « Permetyls® », les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination « Shell Solt® » par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 6 centistokes (6.10⁻⁶ m²/s), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, l'heptaméthyl éthyl trisiloxane, l'heptaméthyl butyl trisiloxane, et leurs mélanges.

On peut également utiliser des solvants organiques volatils notamment fluorés tels que le nonafluorométhoxy butane ou le perfluorométhylcyclopentanc.

Avantageusement, le ou les huile(s) volatile(s) est ou sont choisie(s) parmi les huiles volatiles hydrocarbonées ayant de 8 à 16 atomes de carbone, telle que l'isododécane, les huiles volatiles siliconées telles que le décaméthyl cyclopentasiloxane (D5), le dodécaméthyl cyclohexasiloxane (D6) et leurs mélanges.

La composition selon l'invention peut également comprendre au moins un composé non volatil, non soluble dans l'eau et liquide à température ambiante, notamment au moins une huile ou solvant organique non volatile, qui peut être en particulier choisie parmi les huiles hydrocarbonées et/ou siliconées et/ou fluorées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société STEARINERIES DUBOIS ou ceux vendus sous les dénominations de Miglyol 810® , 812® et 818® par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ; et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans la composition de l'invention sont notamment des huiles fluorosiliconées, des polyéthers fluorés, des silicones fluorées telles que décrit dans le document EP-A-847752.

Le teneur en huile ou solvant organique volatile dans une composition selon l'invention peut varier de 20 à 80 % en poids, en particulier de 30 à 70 % en poids, et mieux de 35 à 60 % en poids par rapport au poids total de la composition.

La teneur en huile ou solvant organique non volatile dans une composition selon l'invention peut varier de 0,01 à 30 % en poids, en particulier de 0,1 à 25 % en poids, et mieux de 0,1 à 20 % en poids par rapport au poids total de la composition.

### EAU ET/OU SOLVANT HYDROSOLUBLE

Les compositions selon l'invention peuvent comprendre une phase aqueuse comprenant de l'eau et/ou au moins un solvant hydrosoluble. Dans ce cas, la teneur en eau et/ou en solvant hydrosoluble est inférieure ou égale à 50 % en poids, de préférence à 40 % en poids, mieux inférieure ou égale à 30 % en poids par rapport au poids total de la composition.

De préférence, sa teneur est inférieure à 20 % en poids, elle peut varier de 0,1 à 20 % et plus particulièrement de 1 à 10 % en poids par rapport au poids total de la composition.

En tout état de cause, la teneur en eau et/ou en solvant(s) hydrosoluble(s) dans la composition selon l'invention est telle que la composition reste sous forme d'une phase continue huileuse, sans inversion de phase.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions selon l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en C₃ et C₄ et les aldéhydes en C₂-C₄.

La phase aqueuse (eau et/ou solvant(s) hydrosoluble(s)) peut être introduite en tant que telle dans la formulation selon l'invention ou y être incorporée par le biais, d'un ou plusieurs ingrédients constituant ladite composition. Ainsi de l'eau peut être notamment introduite dans la composition par le biais de l'introduction de latex ou de pseudolatex, c'est-à-dire de dispersion aqueuse de particules de polymère.

On parle de composition « anhydre » lorsque la teneur en eau et/ou solvant(s) hydrosoluble(s) dans la composition est inférieure à 5 % en poids par rapport au poids total de la composition. Ce type de composition fait également partie de l'invention.

Tous les composants décrits ci-après, qui peuvent être compris dans la composition selon la présente invention seront bien entendu incorporés dans des teneurs telles que leur présence n'affecte pas ou substantiellement pas l'effet recherché, à savoir le maintien d'une tenue à l'eau tout en démontrant une aptitude au démaquillage améliorée.

### AGENT STRUCTURANT

La composition selon l'invention peut comprendre au moins un agent structurant de la phase huileuse ou solvant organique choisi parmi les cires, les polymères semi-cristallins, les gélifiants lipophiles et leurs mélanges.

L'agent structurant peut représenter de 0,1 à 80 % en poids par rapport au poids total de la composition, de préférence de 0,5 à 50 % et de façon encore plus préférée de 1 à 40 % en poids. La quantité en structurant huileux peut être ajustée par l'homme du métier en fonction du propriétés de structuration desdits agents.

### Cire(s)

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

Les cires pouvant être utilisées dans les compositions selon l'invention présentent généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu sous la dénomination TA-TX2i® par la société RHEO, équipé d'un mobile en inox en forme de cylindre d'un diamètre de 2 mm en mesurant l'évolution de la force (force de compression ou force d'étirement) (F) en fonction du temps, pendant l'opération suivante :

Le mobile est déplacé à la vitesse de 0,1 mm/s puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,1 mm/s. Pendant le temps de relaxation, la force (force de compression) décroît fortement jusqu'à devenir nulle puis lors du retrait du mobile, la force (force d'étirement) devient négative pour ensuite croître à nouveau vers la valeur 0. La dureté correspond à la force de compression maximale mesurée entre la surface du mobile et la cire au moment de leur mise en contact. La valeur de cette force est exprimée en MPa.

Pour effectuer la mesure de la dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté.

A titre illustratif des cires convenant à l'invention, on peut notamment citer les cires hydrocarbonées comme la cire d'abeilles, la cire de lanoline, les cires d'insectes de Chine, la cire de sumac, les paraffines, certaines cires de polyéthylène et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50® , l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone, les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Selon un mode de réalisation particulier, les compositions selon l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les fibres kératiniques, ayant une bonne accroche sur les fibres kératiniques et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination TA-TX2i® par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en C₂₀-C₄₀ (le groupe alkyle comprenant de 20 à 40 atomes de carbone), seul ou en mélange, en particulier un 12-(12'-hydroxystéaryloxy)stéarate d'alkyle en C₂₀-C₄₀, de formule (1) : 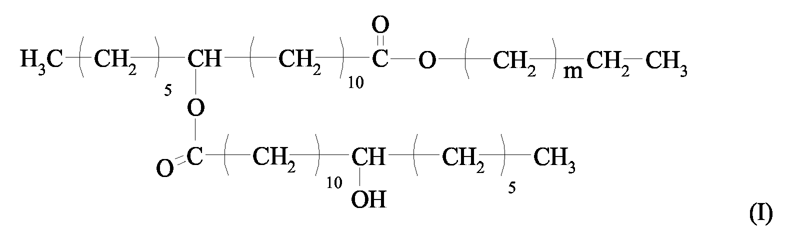 dans laquelle m est un entier allant de 18 à 38, ou un mélange de composés de formule (II).

Une telle cire est notamment vendue sous les dénominations Kester Wax K 82 P® et Kester Wax K 80 P® par la société KOSTER KEUNEN.

Les cires citées ci-dessus présentent généralement un point de fusion commençante inférieur à 45 °C.

Dans la présente invention, on peut également utiliser des cires fournies sous forme de petites particules ayant une dimension exprimée en diamètre « effectif » moyen en volume D[4,3] de l'ordre de 0,5 à 30 micromètres, en particulier de 1 à 20 micromètres, et plus particulièrement de 5 à 10 micromètres, désignées par la suite par l'expression « micro cires ». A des fins de distinction, les cires mises en oeuvre selon l'invention sous forme de fragments de dimension plus élevée sont par la suite désignées par l'expression « cires de type traditionnel ».

Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200® , 220® , 220L® et 250S® par la société MICRO POWDERS et les micro cires de polytétrafluoroéthylène telles que celles commercialisées sous les dénominations de Microslip 519® et 519 L® par la société MICRO POWDERS.

Dans la composition selon l'invention, on peut bien entendu utiliser un mélange de cires et notamment utiliser une ou plusieurs cires de type traditionnel telles que notamment une cire collante et/ou une cire à point de fusion commençante supérieur ou égal à 45 °C, et une ou plusieurs cires dites micro cires. La composition selon l'invention peut comprendre une teneur en cires allant de 0,1 à 70 % en poids par rapport au poids total de la composition, en particulier elle peut en contenir de 0,5 à 50 %, plus particulièrement de 1 à 30 %.

### Polymères semi-cristallins

On entend par polymère des composés comportant au moins deux motifs, de préférence au moins 3 motifs et plus spécialement au moins 10 motifs de répétition. Par « polymère semi-cristallin », on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par « séquence », on entend généralement au moins 5 motifs de répétition identiques. La ou les séquences cristallisables sont alors de nature chimique différente de la ou des séquences amorphes.

Le polymère semi-cristallin a une température de fusion supérieure ou égale à 30°C (notamment allant de 30°C à 80°C), de préférence allant de 30°C à 60°C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins auxquelles s'applique l'invention présentent une masse moléculaire moyenne en nombre supérieure ou égale à 1 000. De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.Par « chaîne ou séquence cristallisable », on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la « chaîne pendante cristallisable » peut être chaîne comportant au moins 6 atomes de carbone.

Le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

De tels polymères sont décrits par exemple dans le document EP 1396259.

A titre d'exemple particulier de polymère semi-cristallin structurant utilisable dans la composition selon l'invention, on peut citer les produits Intelimer® de la société Landec décrits dans la brochure « Intelimer® polymers », Landec IP22 (Rev. 4-97). Ces polymères sont sous forme solide à température ambiante (25°C). Ils sont porteurs de chaînes latérales cristallisables et présentent la formule X précédente.

### Gélifiants lipophiles

Les gélifiants utilisables dans les compositions selon l'invention peuvent être des gélifiant lipophiles organiques ou minéraux, polymériques ou moléculaires.

Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6^{ème} édition, 1995). Elles sont par exemple commercialisées sous les références Aerosil R812® par la société DEGUSSA, CAB-O-SIL TS-530® par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6^{ème} édition, 1995). Elles sont par exemple commercialisées sous les références Aerosil R972® , et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6® , KSG16® et de KSGI8® par la société SHIN-ETSU, de Trefil E-505C® et Trefil E-506C® par la société DOW-CORNING, de Gransil SR-CYC® , SR DMF10® , SR-DC556® , SR 5CYC gel® , SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethoccl® par la société DOW CHEMICAL ; les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

### POLYMERE FILMOGENE

La composition selon l'invention peut comprendre selon un mode de réalisation particulier au moins un polymère filmogène.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente invention, on entend par « polymère filmogène », un polymère apte à former à lui seul ou en présence d'un agent auxiliaire de filmification, un film macroscopiquement continu et adhérent sur les fibres kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

Parmi les polymères filmogènes utilisables dans la composition de la présente invention, on peut citer les polymères synthétiques, de type radicalaire ou de type polycondensat, les polymères d'origine naturelle, et leurs mélanges.

Ces polymères filmogènes sont de préférence distincts du polyélectrolyte précédemment défmi.

### Polymères liposolubles

Selon une variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). Par « phase grasse liquide », on entend, au sens de l'invention, une phase grasse liquide à température ambiante (25°C) et pression atmosphérique (760 mm de Hg, soit 105 Pa), composée d'un ou plusieurs corps gras liquides à température ambiante, tels que les huiles décrites plus haut, généralement compatibles entre eux.

De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de « MDTQ », la résine étant décrite en fonction des différentes unités monomèriques siloxane qu'elle comprend, chacune des lettres « MDTQ » caractérisant un type d'unité.

A titre d'exemples de résines polymethylsilsesquioxanes commercialement disponibles, on peut citer celles qui sont commercialisés :
- par la société Wacker sous la référence Resin MK tels que la Belsil PMS MK:

- par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination «KF-7312J» par la société Shin-Etsu, « DC 749 », « DC 593 » par la société Dow Corning.

On peut également utiliser les polyamides siliconés du type polyorganosiloxane tels que ceux décrits dans les documents US-A-5 874 069, US-A-5,919,441, US-A-6,051,216 et US-A-5,981,680.

Ces polymères siliconés peuvent appartenir aux deux familles suivantes :
1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1411069 ou WO 04/028488.

### Polymère hydrosoluble

Selon une autre variante de réalisation de la composition selon l'invention, le polymère filmogène peut être un polymère hydrosoluble et peut être présent dans une phase aqueuse de la composition ; le polymère est donc solubilisé dans la phase aqueuse de la composition. Comme exemples de polymères filmogènes hydrosolubles, on peut citer :
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :

- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels les chondroïtines sulfate, et leurs mélanges.

### Polymères d'origine naturelle

Les polymères d'origine naturelle, éventuellement modifiés, peuvent être choisis parmi la résine shellac, la gomme de sandaraque, les dammars, les élémis, les copals, les polymères cellulosiques, et leurs mélanges.

### Polymères sous forme dispersée

Le polymère filmogène peut être présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

### a) Dispersion aqueuse

Comme dispersion aqueuse de polymère filmogène, on peut utiliser les dispersions acryliques vendues sous les dénominations Neocryl XK-90® , Neocryl A-1070® , Neocryl A-1090® , Neocryl BT-62® , Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer Allianz Opt® par la société Rohm and Haas ou encore les dispersions aqueuses de polyuréthane vendues sous les dénominations Neorez R-981® et Neorez R-974® par la société AVECIA-NEORESINS, les Avalure UR-405® , Avalure UR-410® , Avalure UR-425® , Avalure UR-450® , Sancure 875® , Sancure 861® , Sancure 878® et Sancure 2060® par la société GOODRICH, Impranil 85® par la société BAYER, Aquamere H-1511® par la société HYDROMER; les sulfopolyesters vendus sous le nom de marque Eastman AQ® par la société EASTMAN CHEMICAL PRODUCTS, les dispersions vinyliques comme le Mexomère PAM® , les dispersions aqueuses de polyvinyl acétate comme le « Vinybrane® » de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références « Hybridur® » par la société AIR PRODUCTS ou « Duromer® » de NATIONAL STARCH, les dispersions type core/shell : par exemple celles commercialisées par la société ATOFINA sous la référence Kynar (core : fluoré-shell : acrylique) ou encore ceux décrits dans le document US 5 188 899 (core ; silice - shell : silicone) et leurs mélanges.

### b) Dispersion non aqueuse

On peut aussi citer les dispersions de particules d'un polymère éthylénique greffé, de préférence acrylique, dans une phase grasse liquide, le polymère éthylénqiue étant avantageusement dispersé en l'absence de stabilisant additionnel en surface des particules telles que décrite notamment dans le document WO 04/055081.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

A ce titre, on peut citer les dispersions acryliques dans l'isododécane comme le Mexomère PAP® par la société CHIMEX

### MATIERE COLORANTE

La composition selon l'invention peut également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de la composition.

### CHARGES

La composition selon l'invention peut en outre comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® commercialisé sous la dénomination Orgasol® par la société ATOCHEM, de poly-β-alanine et de polyéthylène, les poudres de polymères de tétrafluoroéthylène comme le Téflon® , la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel® par la société NOBEL INDUSTRIE, les poudres acryliques telles que celles commercialisées sous la dénomination Polytrap® par la société DOW CORNING, les particules de polyméthacrylate de méthyle et les microbilles de résine de silicone (Tospearls® de TOSHIBA, par exemple), le carbonate de calcium précipité, le carbonate et l'hydro-carbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium, les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel® 007WUpar la Société AKZO NOBEL.

Les charges peuvent représenter de 0,1 à 25 %, en particulier de 1 à 20 % en poids par rapport au poids total de la composition.

### FIBRES

La composition selon l'invention peut en outre comprendre des fibres qui permettent notamment une amélioration de l'effet allongeant lorsque la composition est un mascara.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 µm à 10 mm, en particulier de 0,1 mm à 5 mm et plus particulièrement de 0,3 mm à 3,5 mm. Leur section peut être comprise dans un cercle de diamètre allant de 2 nm à 500 µm, en particulier allant de 100 nm à 100 µm et plus particulièrement de 1 µm à 50 µm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon® ) ou les fibres rigides telles que les fibres de polyimide-amide comme celles vendues sous les dénomination KERMEL® , KERMEL TECH® par la société RHODIA ou de poly-(p-phénylène-téréphtalamide) (ou d'aramide) notamment vendues sous la dénomination Kevlar ® par la société DUPONT DE NEMOURS.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

### ACTIFS COSMETIQUES

La composition selon l'invention peut, de plus, comprendre tous les ingrédients classiquement utilisés en cosmétique. Ces ingrédients peuvent notamment être choisis parmi les polymères, notamment les polymères fixants ; les agents conditionneurs de cheveux ; les opacifiants ; les parfums ; les épaississants ; les gélifiants ; les colorants capillaires ; les résines de silicone ; les gomme de silicone ; les conservateurs ; les antioxydants, les actifs cosmétiques ; les filtres solaires ; les agents de stabilisation du pH ; les vitamines ; les hydratants ; les agents antitranspirants ; les agents déodorants ; les composés autobronzants et leurs mélanges. Les quantités de ces différents ingrédients sont celles classiquement utilisées dans les domaines concernés et par exemple de 0,01 à 20 % du poids total de la composition.

### FORMULATION

La composition selon l'invention peut se présenter sous forme liquide, pâteuse, solide, de mousse ou de spray. Il peut s'agir d'une composition anhydre.

La composition selon l'invention peut être utilisée pour le maquillage de la peau, des lèvres et/ou des fibres kératiniques d'être humain. La composition trouve donc une application particulière comme composition de maquillage du corps ou du visage telle que fond de teint, rouge à lèvres, soin pour lèvres, vernis à ongles, soin des ongles, mascara, eye-liner ; composition capillaire telle que composition de coloration des cheveux ; composition de protection solaire ; composition à rincer à appliquer avant ou après une coloration, une décoloration, une permanente ou un défrisage ou encore entre les deux étapes d'une permanente ou d'un défrisage ; composition capillaire pour le maintien de la coiffure telle qu'une laque, un gel, une mousse ou un spray de coiffage.

Selon un aspect préféré de l'invention, la composition est sous la forme de rouges à lèvres ou de produits du teint, notamment du type fond de teint, ou encore de mascara.

### PROCEDE

Dans tous les cas, les compositions selon l'invention peuvent être préparées selon des méthodes connues de l'homme du métier.

Dans le cas d'une composition destinée au maquillage des fibres kératiniques, le procédé de préparation des compositions selon l'invention dépend du type de mascara recherché. Il dépend encore en particulier de la nature de la ou des cires utilisées.

La présente invention a encore pour objet un procédé de maquillage et/ou de soin non thérapeutique des fibres kératiniques, dans lequel on applique sur lesdites fibres kératiniques notamment les cils, une composition non rincée telle que définie précédemment.

Les compositions de l'invention peuvent être en particulier appliquées sur les cils, à l'aide d'une brosse ou d'un peigne.

L'effet épaississant du maquillage, utilisant la composition de l'invention peut par ailleurs être renforcé en sélectionnant tout particulièrement le dispositif pour l'application de ladite composition.

En l'occurrence, il est particulièrement avantageux de procéder, dans le cas du maquillage des cils, à l'application de ladite composition avec une brosse de maquillage telle que décrite dans les brevets FR 2 701 198, FR 2 605 505, EP 792 603 et EP 663 161.

La composition selon l'invention peut être conditionnée dans un récipient délimitant au moins un compartiment qui comprend ladite composition, ledit récipient étant fermé par un élément de fermeture.

Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par « encliquetage » on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

Le contenu des brevets ou demandes de brevets cités précédemment sont incorporés par référence dans la présente demande.

Les exemples qui suivent sont présentés à titre illustratif et non limitatif de l'invention. Sauf indication contraire, les quantités sont données en gramme.

Les tests suivants ont été utilisés pour l'évaluation de la résistance à l'eau.

### I - Test d'évaluation de la résistance à l'eau et du démaquillage par immersion

### Eprouvette et maquillage

On a réalisé des éprouvettes de faux-cils avec des cheveux caucasiens raides noirs de 19 mm de longueur de frange. On a monté lesdites franges entre deux plaques de 30 mm sur 30 mm.

On a maquillé les cheveux avec la composition testée en effectuant trois fois dix passages espacés de deux minutes à l'aide d'une brosse à mascara.

On a laissé séché pendant une heure à température ambiante (25 °C).

### Descriptif du test

On a immergé l'éprouvette dans l'eau pendant quatre minutes. On a observé visuellement l'apparition d'une dégradation du film (gonflement, irrégularité, décrochement, etc.) et l'on a noté le temps correspondant au début de ce phénomène.

Le temps *t* correspond au temps à partir duquel le film de mascara sur l'éprouvette commence à s'altérer visuellement.

### II - Test d'évaluation du démaquillage sur coton sec

L'éprouvette utilisée pour le test I décrit ci-dessus a ensuite été pincée dans un coton pendant dix secondes puis l'on a tiré le coton pour la démaquiller. On a alors noté l'efficacité du démaquillage en évaluant la quantité de mascara sur le coton et sur les cils.

### Exemple 1 : Mascara à base de cire

### Composition A :

| | % en poids |
|---|---|
| Micro-cire de Carnauba (point de fusion : 83-86 °C) | 18,30 |
| Condensat diacide en C36 hydrogéné/éthylène diamine, estérifié par alcool stéarylique protégé (Uniclear 100 VG d'Arizona) | 1,50 |
| Hectorite modifiée distérayl diméthyl ammonium | 3,60 |
| Oxyde de fer noir | 3,80 |
| Bleu outremer | 1,90 |
| Eau | 7,30 |
| Copolymère acétate de vinyle/stéarate d'allyle (65/35) | 6,50 |
| Carbonate de propylène | 1,20 |
| Cire d'abeille blanche | 13,00 |
| Isododécane | qsp 100 |
| P-hydroxybenzoate de propyle | 0,20 |
| Stéarate d'oligomère d'acide 12-hydroxy-stéarique (Solsperse 21000 d' Uniquema) | 0,60 |
| Micro-cire de paraffine | 6,50 |

Des compositions comprenant la composition A ainsi qu'au moins un polyélectrolyte et au moins un tensioactif de HLB supérieur ou égal à 6 à 25 °C sont préparées par mélange de ces composants dans les proportions figurant dans le tableau I ci-après.

Les résultats de tenue à l'eau et de comportement du démaquillage sont rassemblés dans le tableau 1 ci-après.

**Tableau I**

| Essais | 1 (comparatif) | 2 (comparatif) | 3 (invention) | 4 (invention) | 5 (invention) |
|---|---|---|---|---|---|
| Composition A | 96 | 100 | 88 | 94 | 88 |
| SIMULGEL 600® ⁽¹⁾ | | | 10 | | 10 |
| COSMEDIA SP® | 4 | | | 4 | |
| POLYSORBATE 80 | | | 2 | 2 | |
| POLYSORBATE 20 | | | | | 2 |
| Comportement dans l'eau après immersion dans l'eau 4 min (Test I) | RAS | | t=30s | t=20s | t= 1min 10s |
| Comportement au démaquillage après 1 tiré/ pincé sur coton sec (test II) | TRES LEGER DEMAQUILLAGE | PAS DE DEMAQUILLAGE | DEMAQUILLAGE TOTAL | DEMAQUILLAGE TOTAL | DEMAQUILLAGE TOTAL |

| | | | | | |
|---|---|---|---|---|---|
| RAS = Rien à signaler | | | | | |

⁽¹⁾Le Simulgel 600® contient :
- 4 % de Copolymère acrylamide/acrylamido-2-méthyl propane sulfonate de sodium,
- 0,7 % de polysorbate 80,
- 2,1 % d'isohéxadécane,
- 2,95 % d'eau,
- 0,25 % de sorbitan oléate.

### Exemple 2 :

### 2.1 Microdispersion de cire de carnauba

On a préparé une microdispersion de cire de carnauba ayant la composition suivante :

| | | |
|---|---|---|
| - Cire de carnauba | | 27,00 g |
| - Monostéarate de glycéryle polyoxyéthyléné (30 OE) (TAGAT S de GOLDSCHMIDT) | | 6,75 g |
| - Ethanol | | 10,00 g |
| - Eau | qsp | 100,00 g |

On a chauffé à 90 °C la cire et le tensioactif en homogénéisant le mélange sous agitation modérée. Puis on a incorporé l'eau chauffée à 90 °C en continuant d'agiter. On a refroidi à température ambiante et ajouté l'éthanol pour obtenir une microdispersion de cire ayant un diamètre moyen de particules d'environ 170 nm.

### 2.2 Mascara à base de cire

| | % en poids |
|---|---|
| - Cire de Candellila esterfiée avec polydiméthylsiloxane polyalcoxyle (Siliconyl candellila wax de Koster Keunen) | 0,92 |
| - Cire de carnauba | 4,37 |
| - Microdispersion de cire de carnauba | 7,00 |
| - Cire d'abeille synthétique | 3,12 |
| - Cire de paraffine | 2,10 |
| - Cire de polyoléfine fortement branchée | 0,10 |
| - Cire de polyéthylène | 1,85 |
| - Monooléate de sorbitan oxyéthyléné (20 OE) (Tween 80 d'Uniquema) | 1,75 |
| - Hectorite modifiée distérayl diméthyl ammonium | 5,80 |
| - Hydroxyéthylcellulose quaternisée par chlorure de 2,3-epoxypropyl triméthyl ammonium | 0,10 |
| - Polylaurate de vinyle (Mexomère PP de Chimex) | 0,75 |
| - Copolymère acétate de vinyle/stéarate d'allyle (65/35) | 2,21 |
| - Polyméthacrylate de sodium dans eau à 25 % en MA (DARVAN N°7 de VANDERBILT) | 1,00 |
| - Copolymère vinyle pyrrolidone / eicosène | 2,00 |
| - Polydiméthylsiloxane oxyéthyléné (20 OE) / oxypropyléné (20 OP) | 0,10 |
| - Polybutène (PM 2060) | 1,00 |
| - Carbonate de propylène | 1,94 |
| - Amidon de riz | 1,50 |
| - Copolymère Acrylamide/AMPS Na dans isohexadécane avec polysorbate 80 (Simulgel 600® de SEPPIC) | 1,40 |
| - Oxyde de fer noir | 4,19 |
| - Eau | 2,89 |
| - Ethanol | 2,00 |
| - Conservateurs | qs |
| - Isododécane | qsp 100 |

Cette formule présente une bonne tenue et se démaquillage aisément avec un démaquillant à phase aqueuse tel que celui commercialisé sous la référence Effacils.

### Exemple 3 : Influence de la présence d'un agent tensioactif non ionique de HLB supérieur ou égal à 6 à 25 °C.

### Composition

| **ESSAIS** | **11** | **12** | **13** | **14** |
|---|---|---|---|---|
| Composition A | 90 | 90 | 88 | 88 |
| Copolymère Acrylamide/AMPS Na dans isohexadécane avec polysorbate 80, Simulgel 600® de SEPPIC | -- | 10 | -- | 10 |
| Glycolate sodique d'amidon réticulé, Primojel de AVEBE | 10 | -- | 10 | -- |
| Mono olétate de sorbitane polyoxyéthyléné (20), Tween 80 V® de UNIQUEMA | -- | -- | 2 | 2 |

### Résultats des tests d'évaluation de la résistance à l'eau et du démaquillage

| **ESSAIS** | **11** | **12** | **13** | **14** |
|---|---|---|---|---|
| Temps correspondant à l'apparition d'une dégradation du film (minutes) (test I) | >4 | 3,5 | 2 | 0,5 |
| Evaluation du démaquillage sur coton (test II) | Pas de trace | Léger démaquillage en grain | Démaquillage partiel | Démaquillage Total en colorant |

La comparaison des essais 11 et 13 ainsi que des essais 12 et 14 montre que la présence de Tween 80 V® améliore très nettement le démaquillage.

## Revendications

1. Composition cosmétique pour le maquillage des matières kératiniques, ladite composition ayant une phase continue huileuse et comportant au moins un polyélectrolyte, au moins un agent tensioactif de HLB supérieur ou égal à 6 à 25 °C et au moins une matière colorante, **caractérisée en ce que** le polyélectrolyte est branché et/ou réticulé et **en ce que** la teneur en eau et/ou en solvant hydrosoluble éventuellement présente est inférieure à 50 % en poids par rapport au poids total de la composition.

2. Composition cosmétique non rincée pour le maquillage et/ou le soin non thérapeutique des fibres kératiniques, ladite composition ayant une phase continue huileuse et comportant au moins un polyélectrolyte et au moins un agent tensioactif de HLB supérieur ou égal à 6 à 25 °C, **caractérisée en ce que** le polyélectrolyte est branché et/ou réticulé et **en ce que** la teneur en eau et/ou en solvant hydrosoluble éventuellement présente est inférieure à 50 % en poids par rapport au poids total de la composition.

3. Composition cosmétique non rincée pour le maquillage et/ou le soin non thérapeutique des fibres kératiniques, ladite composition ayant une phase continue huileuse et comportant au moins un polyélectrolyte et au moins un agent tensioactif de HLB supérieur ou égal à 6 à 25 °C, **caractérisée en ce que** le polyélectrolyte est choisi parmi un copolymère acrylamide/acide 2-méthyl 2-[(1-oxo-2-propényl)amino]1-propanesulfonique, un glycolate d'amidon réticulé sous forme de poudre, un polyacrylate, un copolymère greffé à base d'amidon, les dérivés ionisables de polysaccharides, les acides polyacryliques, les copolymères acide polyacryliques acrylates d'alkyle, l'AMPS (Acide polyacrylamidométhyl propane sulfonique neutralisé partiellement à l'ammoniaque et hautement réticulé), les copolymères AMPS/méthacrylates d'alkyle polyoxyéthylénés (réticulés ou non), et un mélange de ces derniers et de préférence parmi un copolymère acrylamide/acide 2-méthyl 2-[(1-oxo-2-propényl)amino]1-propanesulfonique, un polyacrylate, et leurs copolymères.

4. Composition selon l'une quelconque des revendications 1 à 3, **caractérisée en ce que** le polyélectrolyte est choisi parmi un copolymère acrylamide/acide 2-méthyl 2-[(1-ono 2-propényl)amino]1-propanesulfonique, un polyacrylate et leurs copolymères.

5. Composition cosmétique selon l'une quelconque des revendications 1 à 4, **caractérisée en ce que** le polyélectrolyte est présent dans une teneur en poids supérieure ou égale à 0,05 % et de façon préférée supérieure ou égale à 0,1 %, par rapport au poids total de la composition, et notamment supérieure ou égale à 0,5 %.

6. Composition selon l'une quelconque des revendications 1 à 5, **caractérisée en ce que** le polyélectrolyte est présent dans la composition cosmétique de préférence dans une teneur allant de 0,05 à 15 % en poids, de façon encore plus préférée de 0,1 à 10 % en poids, et mieux encore de 0,5 à 5 % en poids par rapport au poids total de la composition.

7. Composition cosmétique selon l'une quelconque des revendications 1 à 6, **caractérisée en ce que** l'agent tensioactif est choisi parmi un agent tensioactif non ionique, un agent tensioactif ionique, un agent tensioactif à caractère mixte ionique et non ionique ou un mélange de ces derniers.

8. Composition cosmétique selon l'une quelconque des revendications 1 à 7, **caractérisée en ce que** l'agent tensioactif est présent en une proportion allant de 0,1 à 30 % en poids, mieux de 0,5 à 15 % en poids et mieux de 1,5 à 10 % en poids par rapport au poids total de la composition.

9. Composition cosmétique selon l'une quelconque des revendications 1 à 8, **caractérisée en ce que** l'agent tensioactif de HLB supérieur ou égal à 6 à 25 °C est choisi parmi les éthers oxyéthylénés et/ou oxypropylénés de glycérol, les éthers oxyéthylénés et/ou oxypropylénés d'alcool gras, les esters d'acide gras et de polyéthylène glycol, les esters d'acide gras et des éthers de glycérol oxyéthylénés et/ou oxypropylénés, les esters d'acide gras et des éthers de sorbitol oxyéthylénés et/ou oxypropylénés, la diméthicone copolyol, la diméthicone copolyol benzoate, les copolymères d'oxyde propylène et d'oxyde d'éthylène, les tensioactifs siliconés, les dérivés d'acides aminés, les sels d'acides gras en C₁₆-C₃₀, les sels d'acides gras polyoxyéthylénés, les esters phosphoriques et leurs sels, les sulfosuccinates, les alkyléthersulfates, les iséthionates, les acylglutamates, les alkyl-imidazolidinium, les sels d'ammonium, et leurs mélanges.

10. Composition cosmétique selon l'une quelconque des revendications 1 à 9, **caractérisée en ce qu'**elle comprend de l'eau et/ou un solvant hydrosoluble dans une teneur inférieure ou égale à 40 % en poids, mieux inférieure ou égale à 30 % en poids et encore mieux à 20 % en poids, en particulier dans une teneur allant de 0,1 à 20 % et plus particulièrement de 1 à 10 % en poids, par rapport au poids total de la composition.

11. Composition cosmétique selon l'une quelconque des revendications 1 à 10, **caractérisée en ce qu'**elle comprend au moins une huile volatile.

12. Composition selon la revendication 11, **caractérisée en ce qu'**elle comprend une quantité d'huile volatile variant de 20 à 80 %, de préférence de 30 à 70 %, et mieux de 35 à 65 % en poids par rapport au poids total de la composition.

13. Composition selon la revendication 11, **caractérisée en ce que** les huiles volatiles peuvent être des huiles hydrocarbonées, des huiles siliconées, des huiles fluorées ou leurs mélanges.

14. Composition selon l'une quelconque des revendications 1 à 13, **caractérisée en ce qu'**elle comprend en outre un agent structurant pouvant être choisi parmi les cires et les polymères semi-cristallins.

15. Composition selon l'une quelconque des revendications 1 à 14, **caractérisée en ce qu'**elle comprend un polymère filmogène.

16. Composition selon l'une quelconque des revendications 2 à 15, **caractérisée en ce qu'**elle contient en outre une matière colorante.

17. Composition selon l'une quelconque des revendications 1 à 16, **caractérisée en ce qu'**elle contient en outre des charges et/ou des fibres.

18. Procédé cosmétique de maquillage des matières kératiniques, **caractérisé en ce qu'**il comprend au moins une étape d'application sur les matières kératiniques, d'une composition telle que définie selon l'une quelconque des revendications 1 et 4 à 17.

19. Procédé cosmétique de maquillage et/ou de soin non thérapeutique des fibres kératiniques, **caractérisé en ce qu'**il comprend au moins une étape d'application sur les fibres kératiniques d'une composition non rincée telle que définie selon l'une quelconque des revendications 2 à 17.

20. Support maquillé comprenant un maquillage susceptible d'être obtenu selon le procédé défini à la revendication 18 ou 19.

21. Utilisation de l'association d'au moins un polyélectrolyte et d'au moins un agent tensioactif de HLB supérieur ou égal à 6 à 25 °C, pour la préparation d'une composition cosmétique pour le maquillage des matières kératiniques à phase continue huileuse, présentant une bonne tenue à l'eau et une facilité de démaquillage.

22. Utilisation de l'association d'au moins un polyélectrolyte et d'au moins un agent tensioactif de HLB supérieur ou égal à 6 à 25 °C, pour la préparation d'une composition cosmétique non rincée pour le maquillage et/ou le soin non thérapeutique des fibres kératiniques à phase continue huileuse, présentant une bonne tenue à l'eau et une facilité de démaquillage.
